# EUROPÄISCHE PATENTANMELDUNG

(11) **EP 3 301 196 A1**
(43) Veröffentlichungstag der Anmeldung: **04.04.2018**
(21) Anmeldenummer: 16191737.2
(22) Anmeldetag: 30.09.2016
(51) Int. Cl.: C12R 1/38, C12R 1/385, C08B 37/00, C12P 19/04, C11D 3/22, A23L 29/256

(54) **MISCHUNG ENTHALTEND RHAMNOLIPIDE, ALGINATE UND PYOVERDIN**

(71) Anmelder: Biotensidon International AG, 6342 Baar (CH)
(72) Erfinder: SHULGA, Oleksandr, 76646 Bruchsal (DE); KARPENKO, Elena, 79005 Lviv (UA); SHCHEHLOVA, Natalia, 79031 Lviv (UA); VILDANOVA, Roza, 81500 Gorodok (UA); KARPENKO, Oleksandr, 76646 Bruchsal (DE); KARPENKO, Ilona, 76646 Bruchsal (DE); PRYSTAI, Mariana, 76646 Bruchsal (DE); JOEGEL, Joerg, 76344 Egenstein-Leo (DE)
(74) Vertreter: Metten, Karl-Heinz

(57) **Zusammenfassung**

Die verlegende Erfindung betrifft eine Mischung, enthaltend Rhamnolipide, Alginate und Pyoverdin. Des Weiteren betrifft die Erfindung einen supramolekularen Rhamnolipid/Alginat-Komplex sowie einen supramolekularen Rhamnolipid/Alginat/Pyoverdin-Komplex. Außerdem betrifft die Erfindung die Verwendung der Findung gemäß Mischung sowie der erfindungsgemäßen supramolekularen Komplexe.

## Beschreibung

Die vorliegende Erfindung betrifft eine Mischung enthaltend Rhamnolipide, Alginate und Pyoverdin. Ferner betrifft die Erfindung einen supramolekularen Rhamnolipid/Alginat-Komplex. Außerdem betrifft die Erfindung einen supramolekularen Rhamnolipid/Alginat/Pyoverdin-Komplex. Des Weiteren betrifft die Erfindung die erfindungsgemäße Mischung, den erfindungsgemäßen supramolekularen Rhamnolipid/Alginat-Komplex und den supramolekularen Rhamnolipid/Alginat/Pyoverdin-Komplex für die Verwendung in der Therapie von Mensch oder Tier. Schließlich betrifft die Erfindung eine pharmazeutische Zusammensetzung enthaltend eine erfindungsgemäße Mischung, einen supramolekularen Rhamnolipid/Alginat-Komplex oder einen supramolekularen Rhamnolipid/Alginat/Pyoverdin-Komplex.

Mikrobiologisch hergestellte Tensidmoleküle, sogenannte Biotenside, gewinnen zunehmend an Bedeutung. Hinsichtlich ihrer Kritischen Mizellbildungskonzentration (CMC) sind sie mit konventionellen nicht-ionischen Tensiden durchaus vergleichbar. Unter Biotenside lassen sich zum Beispiel Glycolipide, Lipopeptide, Lipoaminosäuren, Lipoproteine, Lipopolysaccharide, Phospholipide, Mono- und Diglyceride sowie Fettsäuren subsumieren. Zur Klasse der Glycolipide gehören die Rhamnolipide, die Sophoroselipide, die tetrahalosehaltigen und andere mycolsäurehaltigen Glycolipide sowie die Cellobiose- und Mannosylerythritollipide. Der hydrophobe Teil der Biotenside basiert üblicherweise auf ungesättigten, gesättigten oder hydroxylierten Fettsäureresten. Geeignete Biotenside lassen sich mikrobiell mit Hilfe von Bakterien und Pilzen herstellen. Bei der biotechnologischen Herstellung von Rhamnolipiden kommen mittlerweile regelmäßig Stämme des gramnegativen Bakteriums *Pseudomonas aeruginosa* zum Einsatz.

In der DE 196 54 942 A1 wird die mikrobielle Herstellung von sogenannten Rhamnolipid/Alginat-Komplexen beschrieben. Beispielsweise wird dort ein zur Erzeugung von Rhamnolipid/Alginat-Komplexen befähigtes Bakterium des Genus *Pseudomonas aeruginosa* ID 96-115 offenbart, das bei der DSMZ-Deutschen Sammlung von Mikroorganismen und Zellkulturen GmbH am 23. Februar 1996 unter der Hinterlegungsnummer DSM 10554 hinterlegt wurde. Die in der DE 196 54 942 A1 gefundenen Rhamnolipid/Alginat-Komplexe sollen sich zur Stabilisierung von Emulsionen eignen. Auch sollen diese Komplexe geeignet sein, durch Umweltkatastrophen mit Öl verschmutzte Tiere von Ölrückständen zu befreien. Hierbei sei von Vorteil, dass die Rhamnolipid/Alginat-Komplexe nicht toxisch sowie unter biologischen Bedingungen abbaubar seien.

Existierende Rhamnolipid-haltige Zusammensetzungen lassen jedoch insbesondere hinsichtlich ihres Eigenschaftsprofils noch Wünsche offen.

Der vorliegenden Erfindung lag daher die Aufgabe zugrunde, das Anwendungsspektrum von Rhamnolipiden zu erweitern sowie den Wirkungsgrad bestehender Rhamnolipid-Systeme zu verbessern.

Demgemäß wurde eine Mischung enthaltend Rhamnolipide, Alginate und Pyoverdin gefunden. Bei Pyoverdin handelt sich um fluoreszierende eisenbindende Peptide enthaltend einen Dihydroxychinolin-Rest. Als Chromophor kommt hierbei regelmäßig (1S)-5-Amino-2,3-dihydro-8,9-dihydroxy-1H-pyrimido[1,2-a]-chinolin-1-carboxylsäure zum Einsatz.

In der erfindungsgemäßen Mischung können Rhamnolipide und Alginate als Rhamnolipid/Alginat-Komplexe und daneben als separate Komponente Pyoverdin vorliegen. Besonders bevorzugt umfassen die erfindungsgemäßen Mischungen alternativ oder zusätzlich einen supramolekularen Rhamnolipid/Alginat/Pyoverdin-Komplex. Die erfindungsgemäßen Mischungen enthaltend supramolekulare Rhamnolipid/Alginat-Komplexe und/oder supramolekulare Rhamnolipid/Alginat/Pyoverdin-Komplexe sind in einer zweckmäßigen Ausführungsform im Wesentlichen frei von Rhamnolipiden, die nicht gebunden in den genannten Komplexen vorliegen. Demgemäß wird die der Erfindung zugrunde liegende Aufgabe ebenfalls gelöst durch einen Rhamnolipid/Alginat/Pyoverdin-Komplex. Auch wird die der Erfindung zu Grunde liegende Aufgabe gelöst durch einen supramolekularen Rhamnolipid/Alginat-Komplex mit einem Molekulargewicht nicht oberhalb von 1 x 10⁶ kDa, vorzugsweise im Bereich von 1 x 10² bis 1 x 10⁶ kDa, besonders bevorzugt im Bereich von 1,0 x 10³ bis 8,0 x 10⁴ kDa und insbesondere im Bereich von 7,5 x 10³ bis 9,0 x 10³ kDa.

In den genannten supramolekularen Komplexen liegen die jeweiligen Bestandteile, Rhamnolipid und Alginat bzw. Rhamnolipid, Alginat und Pyoverdin, zumindest in Teilen miteinander verbunden vor. Supramolekulare Komplexe im Sinne der vorliegenden Erfindung umfassen zwei oder mehr Moleküle oder Ionen, die nicht über kovalente Bindungen, sondern über andere Kräfte wie Wasserstoffbrückenbindungen, Ionenpaar-Bildung, Säure/Base-Wechselwirkungen, Metall/Ligand-Wechselwirkungen, Van-der-Waals-Kräfte und/oder hydrophobe Wechselwirkungen zusammengehalten werden.

Rhamnolipid/Alginat-Komplexe, insbesondere die erfindungsgemäßen Rhamnolipid/Alginat-Komplexe, können wie auch die erfindungsgemäßen Rhamnolipid/Alginat/Pyoverdin-Komplexe über ein oder mehrere Rhamnolipid-Einheiten und/oder über ein oder mehrere Alginat-Einheiten verfügen. Auch können in einem Rhamnolipid/Alginat/Pyoverdin-Komplex ein oder mehrere Pyoverdin-Einheiten, ein oder mehrere Rhamnolipid-Einheiten und/oder über ein oder mehrere Alginat-Einheiten zugegen sein. In einer besonders bevorzugten Ausgestaltung verfügt der erfindungsgemäße Rhamnolipid/Alginat/Pyoverdin-Komplex über drei Rhamnolipid-Einheiten, eine Alginat-Einheit und eine Pyoverdin-Einheit.

In einer bevorzugten Ausführungsform der erfindungsgemäßen Mischung sowie der erfindungsgemäßen Rhamnolipid/Alginat-Komplexe und der erfindungsgemäßen Rhamnolipid/Alginat/Pyoverdin-Komplexe ist vorgesehen, dass in dem, insbesondere erfindungsgemäßen, supramolekularen Rhamnolipid/Alginat-Komplex 60 bis 95 Gew.-% und vorzugsweise 80 bis 90 Gew.-% Rhamnolipid sowie 5 bis 40 Gew.-% und vorzugsweise 10 bis 20 Gew.-% Alginat vorliegen.

Die Rhamnolipid liegen in den erfindungsgemäßen Mischungen und/oder in den erfindungsgemäßen Rhamnolipid/Alginat-Komplexen und/oder in den erfindungsgemäßen Rhamnolipid/Alginat/Pyoverdin-Komplexen vorzugsweise als Mono- und/oder Di-Rhamnolipide vor. Bevorzugt sind solche erfindungsgemäßen Mischungen in denen gleichzeitig Mono- und Di-Rhamnolipide zugegen sind. Besonders zufriedenstellende Resultate stellen sich auch dadurch ein, dass die Mono- und/oder Di-Rhamnolipide jeweils ein oder zwei β-Hydroxyfettsäurereste, vorzugsweise mit derselben Kettenlänge, aufweisen. Hierbei ist vorzugsweise vorgesehen, dass die β-Hydroxyfettsäure-Einheiten auf C₈- bis C₁₆-Fettsäuren, vorzugsweise C₈- bis C₁₂- Fettsäuren, enthaltend keine, eine oder zwei Doppelbindungen basieren. Ganz besonders bevorzugt kommen in den Rhamnolipiden der erfindungsgemäßen Mischungen beta-Hydroxyfettsäuren-Einheiten zum Einsatz, die über keine Doppelbindung verfügen, beispielsweise beta-Hydroxydecansäure (auch kurz 3-OH-C10 oder C10 genannt). Besonders geeignete Rhamnolipide umfassen demgemäß Mono-Rhamnolipid-C10 (auch RL1 genannt), Di-Rhamnolipid-C10 (auch RL2 genannt), Mono-Rhamnolipid-C10C10 (auch RL 3 genannt) und/oder Di-Rhamnolipid-C10C10 (auch RL4 genannt). Solche erfindungsgemäßen Mischungen haben sich hierbei als sehr zweckmäßig erwiesen, in denen RL3 und RL4 enthalten sind, insbesondere als Hauptbestandteile der in der Mischung oder den erfindungsgemäßen Rhamnolipid/Alginat-Komplexen oder den erfindungsgemäßen Rhamnolipid/Alginat/Pyoverdin-Komplexen vorliegenden Rhamnolipide oder als ausschließliche Rhamnolipid-Komponenten. Demgemäß umfassen die erfindungsgemäßen Mischungen sowie die erfindungsgemäßen Rhamnolipid/Alginat-Komplexe und die erfindungsgemäßen Rhamnolipid/Alginat/Pyoverdin-Komplexe vorteilhafter Weise Rhamnolipide, die aus zwei Rhamnose-Einheiten und zwei Caprinsäureeinheiten gebildet sind. Besonders vorteilhaft liegen diese Rhamnolipide als Hauptkomponente der Rhamnolipide in den erfindungsgemäßen Mischungen vor.

In einer bevorzugten Ausgestaltung der erfindungsgemäßen Mischung ist vorgesehen, dass das Gewichtsverhältnis von Mono-Rhamnolipiden und Di-Rhamnolipiden in den, insbesondere erfindungsgemäßen, supramolekularen Rhamnolipid/Alginat-Komplexen und/oder in den supramolekularen Rhamnolipid/Alginat/Pyoverdin-Komplexen im Bereich von 9:1 bis 0,5:5 und bevorzugt im Bereich von 9:1 bis 1:1 liegt.

Besonders vorteilhafte Eigenschaften lassen sich in verlässlicher Weise auch mit solchen erfindungsgemäßen Mischungen erhalten, in denen neben dem, insbesondere erfindungsgemäßen, supramolekularen Rhamnolipid/Alginat-Komplex und/oder dem supramolekularen Rhamnolipid/Alginat/Pyoverdin-Komplex freies Alginat vorliegt, vorzugsweise nicht oberhalb von 40 Gew.-% und besonders bevorzugt nicht oberhalb von 20 Gew.-%, beispielsweise im Bereich von 5 bis 18 Gew.-%, jeweils bezogen auf das Gesamtgewicht der supramolekularen Rhamnolipid/Alginat-Komplexe und der supramolekularen Rhamnolipid/Alginat/Pyoverdin-Komplexe.

Besonders bevorzugt sind auch solche erfindungsgemäßen Mischungen, in denen zusätzlich zu dem supramolekularen, insbesondere erfindungsgemäßen, Rhamnolipid/Alginat-Komplex und/oder dem supramolekularen Rhamnolipid/Alginat/Pyoverdin-Komplex freies Pyoverdin vorliegt, vorzugsweise in einer Menge im Bereich von 0,01 bis 5 Gew.-% und besonders bevorzugt im Bereich von 0,1 bis 2,0 Gew.-%, jeweils bezogen auf das Gesamtgewicht der supramolekularen Rhamnolipid/Alginat-Komplexe und der supramolekularen Rhamnolipid/Alginat/Pyoverdin-Komplexe.

Auch haben sich solche erfindungsgemäßen Mischungen bzw. erfindungsgemäßen supramolekularen Rhamnolipid/Alginat/Pyoverdin-Komplexe als sehr zweckmäßig erwiesen, bei denen der supramolekulare Rhamnolipid/Alginat/Pyoverdin-Komplex 55 bis 94,99 Gew.-% vorzugweise 78 bis 94,9 Gew.% und besonders bevorzugt 80,0 bis 90,0 Gew.-%, an Rhamnolipiden, 50 bis 40 Gew.-%, vorzugsweise 5 bis 20 Gew.-% an Alginaten, und 0,01 bis 5 Gew.-%, vorzugsweise 0,1 bis 2 Gew.-%, an Pyoverdin enthält.

In einer zweckmäßigen Ausgestaltung zeichnet sich die erfindungsgemäßen Mischung oder die erfindungsgemäßen Rhamnolipid/Alginat-Komplexe und/oder die erfindungsgemäßen Rhamnolipid/Alginat/Pyoverdin-Komplexe dadurch aus, dass diese Di-Rhamnolipide und Mono-Rhamnolipide enthält, wobei darin 60 bis 90 Gew.-%, insbesondere 85,0 bis 90,0 Gew.-%, Di-Rhamnolipide, insbesondere di-RL-C10C10, und 10 bis 40 Gew.-%, insbesondere 10 bis 15 Gew.-%, Mono- Rhamnolipide, insbesondere mono-RL-C10C10, jeweils bezogen auf das Gesamtgewicht der supramolekularen Rhamnolipid/Alginat-Komplexe und/oder der supramolekularen Rhamnolipid/Alginat/Pyoverdin-Komplexe, vorliegen. Das Gewichtsverhältnis der genannten di-Rhamnolipide zu den mono-Rhamnolipiden ist dabei vorzugsweise größer 6:4.

Geeignete erfindungsgemäße Mischungen sowie geeignete erfindungsgemäße Rhamnolipid/Alginat-Komplexe und geeignete erfindungsgemäße Rhamnolipid/Alginat/Pyoverdin-Komplexe erhält man z.B. auf biotechnologischem Wege durch Verwendung eines Stamms des Bakteriums *Pseudomonas aeruginosa,* wobei *Pseudomonas aeruginosa* JRV-L, *Pseudomonas aeruginosa* PAO1, *Pseudomonas aeruginosa* ATCC27853 und *Pseudomonas aeruginosa* Pa6 besonders bevorzugt sind.

Geeignete Nährmedien können dabei Harnstoff (beispielsweise im Bereich von 0,5 bis 3 g/Liter, vorzugsweise im Bereich von 1 bis 2 g/Liter), Kaliumdihydrogenphosphat (beispielsweise im Bereich von 0,5 bis 3 g/Liter, vorzugsweise im Bereich von 1 bis 2 g/Liter), Dikaliumhydrogenphosphat (beispielsweise im Bereich von 0, 2 bis 2,5 g/Liter, vorzugsweise im Bereich von 0,8 bis 1,6 g/Liter), Magnesiumsulfat (beispielsweise im Bereich von 0,01 bis 1 g/Liter, vorzugsweise im Bereich von 0,1 bis 0, 5 g/Liter), Hefeextrakt (beispielsweise im Bereich von 0,1 bis 1,5 g/Liter, vorzugsweise im Bereich von 0,2 bis 1,0 g/Liter) und eine Kohlenstoffquelle, beispielsweise Glucose, Glycerin und/oder pflanzliche Öle (beispielsweise im Bereich von 5 bis 30 g/Liter, vorzugsweise im Bereich von 10 bis 24 g/Liter) enthalten.

Erfindungsgemäße Mischungen sowie erfindungsgemäße Rhamnolipid/Alginat-Komplexe und erfindungsgemäße Rhamnolipid/Alginat/Pyoverdin-Komplexe sind insbesondere erhältlich mittels Fermentation in Gegenwart von *Pseudomonas aeruginosa* JRV-L, *Pseudomonas aeruginosa* PAO1, *Pseudomonas aeruginosa* ATCC27853 und/oder *Pseudomonas aeruginosa* Pa6 und Acidifizierung des Überstands. Vorzugsweis wird der pH-Wert beim Acidifizierungsschritt auf Werte in dem Bereich von 1 bis 4 eingestellt, wobei regelmäßig ein Wert im Bereich von 1,0 bis 2,0 verwendet wird. Im Zuge der geschilderten Verringerung des pH-Wertes fallen die erfindungsgemäße Mischung, der erfindungsgemäße Rhamnolipid/Alginat-Komplex und/oder der erfindungsgemäße Rhamnolipid/Alginat/Pyoverdin-Komplex aus. Der erhaltene Niederschlag kann weiteren Aufreinigungsschritten unterzogen werden. Dies ist für den Erhalt der erfindungsgemäßen Wirkungen jedoch nicht zwingend erforderlich. Eine Aufkonzentration des zellfreien Überstands kann auch mittels Sprühtrocknung, Vakuumverdampfung, Membrantechnologie oder Lyophilisieren vorgenommen werden. Unter den vorangehend genannten Möglichkeiten ist die Sprühtrocknung bevorzugt. Durch die beschriebene Aufreinigung kann der Rhamnolipid/Alginat-Komplex sowie gegebenenfalls auch der Rhamnolipid/Alginat/Pyoverdin-Komplex auch in kristalliner Form erhalten werden.

In einer besonders zweckmäßigen Ausgestaltung stellt die erfindungsgemäße Mischung den abgetrennten, gegebenenfalls aufgereinigten, Feststoffanteil aus der fermentativen Herstellung der Mischung, vorzugsweise in Gegenwart von *Pseudomonas aeruginosa,* insbesondere von *Pseudomonas aeruginosa* JRV-L, *Pseudomonas aeruginosa* PAO1, *Pseudomonas aeruginosa* ATCC27853 und/oder *Pseudomonas aeruginosa* Pa6, dar oder ist hierin enthalten. Des Weiteren kann ein zellfreier Kulturüberstand bis zum pulverförmigen Produkt getrocknet werden. In der Regel wird nach der fermentativen Herstellung ein zellfreier Überstand gewonnen, welcher azidifiziert wird, vorzugsweise auf einen pH-Wert im Bereich von 2 bis 4, besonders bevorzugt im Bereich von 1 bis 2, beispielseise auf etwa 2,0.

In einer Ausführungsform wird die Fermentationsbrühe einer sogenannten Mikrofiltration mittels Membranfiler unterworfen, um Zellbestandteile abzutrennen. Die Membranfilter basieren regelmäßig auf keramischen oder polymeren Materialien, beispielsweise auf Polysulfonen. Geeignete Porengrößen dieser Membranfilter für die Mikrofiltration liegen bei 100 nm und darüber, vorzugsweise im Bereich von 0,1 bis 0,2 µm, beispielsweise bei etwa 0,1 µm. Das nach dem Filtrationsschritt erhaltene System stellt im Allgemeinen eine klare Flüssigkeit dar. Diese hat regelmäßig einen pH-Wert im Bereich von 8 bis 9. In diesem wässrigen System liegen neben der erfindungsgemäßen Mischung bzw. dem erfindungsgemäßen Rhamnolipid/Alginat-Komplex und/oder dem erfindungsgemäßen Rhamnolipid/Alginat/Pyoverdin-Komplex häufig auch noch Mineralsalze und gegebenenfalls organische Bestandteile wie Glycerin vor. Dieses wässrige System kann bereits als solches für die in dieser Anmeldeschrift genannten erfindungsgemäßen Verwendungen genutzt werden. Selbstverständlich ist es möglich, das nach der Mikrofiltration erhaltene wässrige System einer sogenannten Ultrafiltration zu unterziehen. Die Porengröße der hierbei jeweils zum Einsatz kommenden Filter liegt üblicherweise zwischen 2 bis 100 nm. Mit Ultrafiltration gelingt es, weitere Bestandteile, beispielsweise Nährsubstrat oder Salze, aus dem wässrigen System enthaltend die erfindungsgemäße Mischung bzw. die erfindungsgemäßen Komplexe zu entfernen. Optional kann in einem weiteren Verfahrensschritt, d.h. nach der Mikro- und/oder Ultrafiltration, das gefilterte wässrige System in die Trockne überführt werden, beispielsweise durch Vakuumverdampfung.

In einer bevorzugten Ausgestaltung liegen in den durch Mikro- und insbesondere durch Ultrafiltration erhaltenen wässrigen Systemen keine freien Rhamnolipide vor. Vielmehr sind diese in den erfindungsgemäßen Komplexen gebunden. Als Alternative zur Isolierung der erfindungsgemäßen Komplexe über das Entfernen von Wasser können die erfindungsgemäßen Komplexe aus den wässrigen Systemen, insbesondere den mikro- und/oder ultrafiltrierten Systemen, durch Verringerung des pH-Wertes auf Werte im Bereich von 1 bis 4, insbesondere im Bereich von 1,0 bis 2,0, ausgefällt werden.

Die der Erfindung zugrunde liegende Aufgabe wird des Weiteren gelöst durch eine erfindungsgemäße Mischung, einen erfindungsgemäßen Rhamnolipid/Alginat-Komplex und einen erfindungsgemäßen Rhamnolipid/Alginat/Pyoverdin-Komplex für die Verwendung in der Therapie von Mensch oder Tier. Insbesondere können die erfindungsgemäße Mischungen, der erfindungsgemäße Rhamnolopid/Alginat-Komplex oder der erfindungsgemäße Rhamnolopid/Alginat/Pyoverdin-Komplex für die Behandlung von dermatologischen Erkrankungen, insbesondere von Schuppenflechte, Ekzemen und/oder Vitiligo, oder für die Wundbehandlung eingesetzt werden.

Des Weiteren können die erfindungsgemäße Mischung, der erfindungsgemäße Rhamnolipid/Alginat-Komplex oder der erfindungsgemäße Rhamnolipid/Alginat/Pyoverdin-Komplex für die Verwendung als oder in einem antimykotischen, antibakteriellen, antiviralen oder antifungischen Präparat eingesetzt werden. Im Sinne der vorliegenden Erfindung sollen unter antimykotischen Präparaten solche verstanden werden, die für die Behandlung von bei Menschen und Tieren auftretenden Pilzerkrankungen geeignet sind, während antifungische Präparate solche sein sollen, die gegen den Pilzbefall von Pflanzen eingesetzt werden können.

Demgemäß können die erfindungsgemäße Mischung oder der erfndungsgemäße supramolekulare Rhamnolipid/Alginat-Komplex oder der supramolekulare Rhamnolipid/Alginat/Pyoverdin-Komplex auch zur Behandlung von Befall mit *Erysiphe necator* (echter Mehltau), *Plasmopara viticola* (falscher Mehltau), *Plasmodiophora, Polymyxa, Spongospora*, *Physoderma, Olpidium, Synchytrium, Rhizophydium*, *Achlya, Aphanomyces*, *Albugo, Peronophthora*, *Pachymetra*, *Pythium, Phytophthora*, *Trachysphaera*, *Basidiophora, Bremia*, *Peronosclerospora, Plasmopara, Pseudoperonospora, Sclerophthora*, *Scierospora, Fusarium,* insbesondere *Fusarium oxysporum TR4, Verticillium, Peronospora, Monilinia* und/oder *Botrytis* eingesetzt werden.

Außerdem können die erfindungsgemäße Mischung, der erfindungsgemäße Rhamnolipid/Alginat-Komplex oder der erfindungsgemäße Rhamnolipid/Alginat/Pyoverdin-Komplex für die Behandlung von Rhinitis oder Nasennebenhöhlenentzündungen eingesetzt werden.

Die der Erfindung zugrunde liegende Aufgabe wird des Weiteren gelöst, durch eine pharmazeutische Zusammensetzung enthaltend eine erfindungsgemäße Mischung oder mindestens einen erfindungsgemäßen Rhamnolipid/Alginat-Komplex oder mindestens einen erfindungsgemäßen Rhamnolipid/Alginat/Pyoverdin-Komplex.

Des Weiteren wird die der Erfindung zugrunde liegende Aufgabe gelöst durch ein Reinigungsmittel, insbesondere fettlösliches Reinigungsmittel, enthaltend eine erfindungsgemäße Mischung, mindestens einen erfindungsgemäßen Rhamnolipid/Alginat-Komplex und/oder mindestens einen erfindungsgemäßen Rhamnolipid/Alginat/Pyoverdin-Komplex.

Auch wird die der Erfindung zugrunde liegende Aufgabe gelöst durch ein Körper-, Haut- oder Haarreinigungs- oder -pflegemittel enthaltend eine erfindungsgemäße Mischung oder einen erfindungsgemäßen Rhamnolipid/Alginat-Komplex und/oder mindestens einen erfindungsgemäßen Rhamnolipid/Alginat/Pyoverdin-Komplex.

Des Weiteren stellt die vorliegende Erfindung ab auf kosmetische Formulierungen, Metalldekontaminationsmittel, insbesondere Schwermetall- und/oder Radionukliddekontaminationsmittel, Dünger, Düngeradditive, Futtermittel, Futtermittelzusatzstoffe, Nahrungsmittel oder Nahrungsmittelzusatzstoffe, Pflanzenschutzmittel, Feuerlöschmittel oder Löschmittzusätze, Korrosionsschutzmittel, Fettlöser und Bohrlochflüssigkeiten enthaltend eine erfindungsgemäße Mischung und/oder mindestens einen erfindungsgemäßen Rhamnolipid/Alginat-Komplex und/oder mindestens einen erfindungsgemäßen Rhamnolipid/Alginat/Pyoverdin-Komplex.

Ferner betrifft die Erfindung behandelte Pflanzensamen bzw. behandeltes Saatgut, erhalten oder erhältlich durch Behandlung von Pflanzensamen mit der erfindungsgemäßen Mischung oder mit mindestens einem erfindungsgemäßen Rhamnolipid/Alginat-Komplex und/oder mit mindestens einem erfndungsgemäßen Rhamnolipid/Alginat/Pyoverdin-Komplex. Hierdurch können z.B. der Krankheitsbefall reduziert werden und/oder das Wachstum positiv beeinflusst werden.

Das Pflanzenschutzmittel kann beispielsweise in Form eines Fungizids oder Insektizids oder auch in Form eines sogenannten Elicitors vorliegen oder als solches dienen. Als Elicitoren werden in der Pflanzenphysiologie Substanzen bzw. Botenstoffe bezeichnet, die Abwehrmechanismen gegen Fraßfeinde und/oder Krankheitserreger induzieren. Der Einsatz bzw. Zusatz der erfindungsgemäßen Mischung oder des erfindungsgemäßen Rhamnolipid/Alginat-Komplexes und/oder des erfindungsgemäßen Rhamnolipid/Alginat/Pyoverdin-Komplexes dient dabei in einer bevorzugten Ausgestaltung zur Aktivierung des Immunsystems von Pflanzen sowie zur Pflanzenstärkung und schützt auch auf dieser Weise vor Schädlingsbefall.

Demgemäß betrifft die Erfindung ebenfalls die Verwendung der erfindungsgemäßen Mischung oder des erfindungsgemäßen Rhamnolopid/Alginat-Komplexes und/oder des erfindungsgemäßen Rhamnolipid/Alginat/Pyoverdin-Komplexes für die Herstellung von Kosmetik, dermatologischen oder pharmazeutischen Formulierungen, von PflanzenschutzFormulierungen oder von Pflege- oder Reinigungsmitteln sowie von Tensidkonzentraten.

Des Weiteren eignen sich die erfindungsgemäßen Mischungen, die erfindungsgemäßen Rhamnolipid/Alginat-Komplexe und/oder die erfindungsgemäßen Rhamnolipid/Alginat/Pyoverdin-Komplexe für den Einsatz bei der Tertiären Erdölförderung, für die Erhöhung der Enzymaktivität sowie für die Bioremediation des Bodens und des Wassers. Des Weiteren hat es sich als zweckmäßig erwiesen, die erfindungsgemäßen Mischungen, die erfindungsgemäßen Rhamnolipid/Alginat-Komplexe und/oder die erfindungsgemäßen Rhamnolipid/Alginat/Pyoverdin-Komplexe für die oder bei der Erdölraffination, beispielsweise um Verunreinigungen wie Phosphorverbindungen (z.B. Phospholipide), Wachse und Teer zu entfernen, oder bei der Raffination von Pflanzenölen einzusetzen. Ferner betrifft die vorliegende Erfindung die erfindungsgemäße Mischung, die erfindungsgemäßen Rhamnolopid/Alginat-Komplexe und/oder die erfindungsgemäßen Rhamnolopid/Alginat/Pyoverdin-Komplexe für die Behandlung von Autoimmunkrankheiten, insbesondere HIV, sowie von Alzheimer.

Die in der vorstehenden Beschreibung und in den Ansprüchen offenbarten Merkmale der Erfindung können sowohl einzeln aus auch in jeder beliebigen Kombination für die Verwirklichung der Erfindung in ihren verschiedenen Ausführungsformen wesentlich sein.

## Patentansprüche

1. Mischung, enthaltend
Rhamnolipid, Alginate und Pyoverdin.

2. Mischung nach Anspruch 1, **dadurch gekennzeichnet, dass**
Rhamnolipid und Alginat zumindest in Teilen miteinander verbunden in Form eines supramolekularen Rhamnolipid/Alginat-Komplexes vorliegen und/oder dass Rhamnolipid, Alginat und Pyoverdin zumindest in Teilen miteinander verbunden in Form eines supramolekularen Rhamnolipid/Alginat/Pyoverdin-Komplexes vorliegen.

3. Mischung nach Anspruch 2, **dadurch gekennzeichnet, dass**
in dem supramolekularen Rhamnolipid/Alginat-Komplex 60 bis 95 Gew.-% und vorzugsweise 80 bis 90 Gew.-% Rhamnolipid sowie 5 bis 40 Gew.-% und vorzugsweise 10 bis 20 Gew.-% Alginat vorliegen.

4. Mischung nach einem der vorangehenden Ansprüche, **dadurch gekennzeichnet, dass** die Rhamnolipide Mono-Rhamnolipide und/oder Di-Rhamnolipide umfassen, insbesondere jeweils enthaltend ein oder zwei β-Hydroxyfettsäure-Einheiten, vorzugweise mit derselben Kettenlänge.

5. Mischung nach Anspruch 4, **dadurch gekennzeichnet, dass**
die β-Hydroxyfettsäure-Einheiten auf C₈- bis C₁₆-Fettsäuren, vorzugsweise C₈- bis C₁₂- Fettsäuren, enthaltend keine, eine oder zwei Doppelbindungen basieren.

6. Mischung nach Anspruch 4 oder 5, **dadurch gekennzeichnet, dass** die β-Hydroxyfettsäure-Einheiten keine, eine oder zwei Doppelbindungen, insbesondere keine Doppelbindungen, enthalten.

7. Mischung nach einem der Ansprüche 4 bis 6, **dadurch gekennzeichnet, dass** das Gewichtsverhältnis von Mono-Rhamnolipiden und Di-Rhamnolipiden in den supramolekularen Rhamnolipid/Alginat-Komplexen und/oder in den supramolekularen Rhamnolipid/Alginat/Pyoverdin-Komplexe im Bereich von 9:1 bis 0,5:5 und bevorzugt im Bereich von 9:1 bis 1:1 liegt.

8. Mischung nach einem der Ansprüche 2 bis 7, **dadurch gekennzeichnet, dass** diese neben dem supramolekularen Rhamnolipid/Alginat-Komplex und/oder dem supramolekularen Rhamnolipid/Alginat/Pyoverdin-Komplex freies Alginat enthält, vorzugsweise nicht oberhalb von 40 Gew.-% und besonders bevorzugt nicht oberhalb von 20 Gew.-%, jeweils bezogen auf das Gesamtgewicht der supramolekularen Rhamnolipid/Alginat-Komplexe und der supramolekularen Rhamnolipid/Alginat/Pyoverdin-Komplexe.

9. Mischung nach einem der Ansprüche 2 bis 8, **dadurch gekennzeichnet, dass** zusätzlich zu dem Rhamnolipid/Alginat-Komplex und/oder dem supramolekularen Rhamnolipid/Alginat/Pyoverdin-Komplex freies Pyoverdin vorliegt, vorzugsweise in einer Menge im Bereich von 0,01 bis 5 Gew.-% und besonders bevorzugt im Bereich von 0,1 bis 2,0 Gew.-%, jeweils bezogen auf das Gesamtgewicht der supramolekularen Rhamnolipid/Alginat-Komplexe und der supramolekularen Rhamnolipid/Alginat/Pyoverdin-Komplexe.

10. Mischung nach einem der Ansprüche 2 bis 9, **dadurch gekennzeichnet, dass** der supramolekulare Rhamnolipid/Alginat/Pyoverdin-Komplex 55 bis 94,99 Gew.-%, vorzugweise 78 bis 94,9 Gew.%, an Rhamnolipiden, 5,0 bis 40 Gew.-%, vorzugsweise 5 bis 20 Gew.-% an Alginaten, und 0,01 bis 5 Gew.-%, vorzugsweise 0,1 bis 2 Gew.-%, an Pyoverdin enthält.

11. Mischung nach einem der vorangehenden Ansprüche, **dadurch gekennzeichnet, dass** die Rhamnolipide gebildet sind aus zwei Rhamnoseeinheiten und zwei Caprinsäureeinheiten.

12. Mischung nach einem der vorangehenden Ansprüche, **dadurch gekennzeichnet, dass** diese als Rhamnolipide, insbesondere ausschließlich, Di-Rhamnolipide und Mono-Rhamnolipide enthält, wobei darin 60 bis 90 Gew.-%, insbesondere 85 bis 90 Gew.-%, Di-Rhamnolipide, insbesondere di-RL-C10C10, und 10 bis 40 Gew.-%, insbesondere 10 bis 15 Gew.-%, Mono-Rhamnolipide, insbesondere mano-RL-C10C10, jeweils bezogen auf das Gesamtgewicht der supramolekularen Rhamnolipid/Alginat-Komplexe und/oder der supramolekularen Rhamnolipid/Alginat/Pyoverdin-Komplexe, vorliegen.

13. Mischung nach einem der vorangehenden Ansprüche, erhalten oder erhältlich durch Verwendung eines Stamms des Bakteriums *Pseudomonas aeruginosa,* insbesondere von *Pseudomonas aeruginosa* JRV-L, *Pseudomonas aeruginosa* ATCC15692, *Pseudomonas aeruginosa* ATCC27853 und/oder *Pseudomonas aeruginosa* Pa6.

14. Mischung nach einem der vorangehenden Ansprüche, erhalten oder erhältlich mittels Fermentation in Gegenwart von *Pseudomonas aeruginosa,* insbesondere von *Pseudomonas aeruginosa* JRV-L, *Pseudomonas aeruginosa* PAO1, *Pseudomonas aeruginosa* ATCC27853 und/oder *Pseudomonas aeruginosa* Pa6 und Azidifizierung des Überstands, bevorzugt auf ein pH-Wert im Bereich von 1 bis 4 und besonders bevorzugt auf einen pH-Wert im Bereich von 1 bis 2,0.

15. Mischung nach einem der vorangehenden Ansprüche, **dadurch gekennzeichnet dass** diese den abgetrennten, gegebenenfalls aufgereinigten, Feststoffanteil aus der fermentativen Herstellung der Mischung in Gegenwart von *Pseudomonas aeruginosa,* insbesondere von *Pseudomonas aeruginosa* JRV-L, *Pseudomonas aeruginosa* PAO1, *Pseudomonas aeruginosa* ATCC27853 und/oder *Pseudomonas aeruginosa* Pa6 darstellt oder hierin enthalten ist.

16. Supramolekularer Rhamnolipid/Alginat-Komplex mit einem Molekulargewicht nicht oberhalb von 1 x 10⁶ kDa, vorzugsweise im Bereich von 1 x 10² bis 1 x 10⁶ kDa, besonders bevorzugt im Bereich von 1,0 x 10³ bis 8,0 x 10⁴ kDa und insbesondere im Bereich von 7,5 x 10³ bis 9,0 x 10³ kDa.

17. Rhamnolipid/Alginat-Komplex nach Anspruch 16, **dadurch gekennzeichnet, dass** in diesem 60 bis 95 Gew.-% und vorzugsweise 80 bis 90 Gew.-% Rhamnolipid sowie 5 bis 40 Gew.-% und vorzugsweise 10 bis 20 Gew.-% Alginat vorliegen.

18. Supramolekularer Rhamnolipid/Alginat/Pyoverdin-Komplex.

19. Rhamnolipid/Alginat/Pyoverdin-Komplex nach Anspruch 18, **dadurch gekennzeichnet, dass**
in diesem 55 bis 94,99 Gew.-%, vorzugweise 78 bis 94,9 Gew.%, an Rhamnolipiden, 5,0 bis 40 Gew.-%, vorzugsweise 5 bis 20 Gew.-%, an Alginaten, und 0,01 bis 5 Gew.-%, vorzugsweise 0.1 bis 2 Gew.-%, an Pyoverdin vorliegen.

20. Mischung nach einem der Ansprüche 1 bis 15 oder supramolekularer Rhamnolipid/Alginat-Komplex nach Anspruch 16 oder 17 oder supramolekularer Rhamnolipid/Alginat/Pyoverdin-Komplex nach Anspruch 18 oder 19 für die Verwendung in der Therapie von Mensch oder Tier.

21. Mischung nach einem der Ansprüche 1 bis 15 oder supramolekularer Rhamnolipid/Alginat-Komplex nach Anspruch 16 oder 17 oder supramolekularer Rhamnolipid/Alginat/Pyoverdin-Komplex nach Anspruch 18 oder 19 für die Behandlung von dermatologischen Erkrankungen, insbesondre von Schuppenflechte, Ekzemen und/oder Vitiligo, oder für die Wundbehandlung.

22. Mischung nach einem der Ansprüche 1 bis 15 oder supramolekularer Rhamnolipid/Alginat-Komplex nach Anspruch 16 oder 17 oder supramolekularer Rhamnolipid/Alginat/Pyoverdin-Komplex nach Anspruch 18 oder 19 für die Verwendung als und/oder in einem antimykotischen, antibakteriellen, antiviralen oder antifungischen Präparat.

23. Mischung nach einem der Ansprüche 1 bis 15 oder supramolekularer Rhamnolipid/Alginat-Komplex nach Anspruch 16 oder 17 oder supramolekularer Rhamnolipid/Alginat/Pyoverdin-Komplex nach Anspruch 18 oder 19 für die Behandlung von Befall mit *Erysiphe necator* (echter Mehltau), *Plasmopara viticola* (falscher Mehltau), *Plasmodiophora, Polymyxa, Spongospora*, *Physoderma*, *Olpidium, Synchytrium, Rhizophydium*, *Achlya, Aphanomyces, Albugo*, *Peronophthora, Pachymetra*, *Pythium, Phytophthora*, *Trackysphaera, Basidiophora*, *Bremia*, *Peronosclerospora*, *Plasmopara, Pseudoperonospora*, *Sclerophthora, Scierospora*, *Fusarium,* insbesondere *Fusarium oxysporum TR4, Verticillium*, *Peronospora, Monilinia* und/oder *Botrytis.*

24. Mischung nach einem der Ansprüche 1 bis 15 oder supramolekularer Rhamnolipid/Alginat-Komplex nach Anspruch 16 oder 17 oder supramolekularer Rhamnolipid/Alginat/Pyoverdin-Komplex nach Anspruch 18 oder 19 für die Behandlung von Rhinitis oder Nasennebenhöhlenentzündungen.

25. Pharmazeutische Zusammensetzung enthaltend die Mischung nach einem der Ansprüche 1 bis 15 oder den supramolekularen Rhamnolipid/Alginat-Komplex nach Anspruch 16 oder 17 oder den supramolekularen Rhamnolipid/Alginat/Pyoverdin-Komplex nach Anspruch 18 oder 19.

26. Reinigungsmittel, insbesondere fettlösliches Reinigungsmittel, enthaltend eine Mischung nach einem der Ansprüche 1 bis 15 oder den supramolekularen Rhamnolipid/Alginat-Komplex nach Anspruch 16 oder 17 oder den supramolekularen Rhamnolipid/Alginat/Pyoverdin-Komplex nach Anspruch 18 oder 19.

27. Körper-, Haut- oder Haarreinigungs- oder -pflegemittel, enthaltend eine Mischung nach einem der Ansprüche 1 bis 15 oder den supramolekularen Rhamnolipid/Alginat-Komplex nach Anspruch 16 oder 17 oder den supramolekularen Rhamnolipid/Alginat/Pyoverdin-Komplex nach Anspruch 18 oder 19.

28. Kosmetische Formulierung, enthaltend
eine Mischung nach einem der Ansprüche 1 bis 15 oder den supramolekularen Rhamnolipid/Alginat-Komplex nach Anspruch 16 oder 17 oder den supramolekularen Rhamnolipid/Alginat/Pyoverdin-Komplex nach Anspruch 18 oder 19.

29. Metalldekontaminationsmittel, insbesondere Schwermetall- und/oder Radionukliddekontaminationsmittel, enthaltend
eine Mischung nach einem der Ansprüche 1 bis 15 oder den supramolekularen Rhamnolipid/Alginat-Komplex nach Anspruch 16 oder 17 oder den supramolekularen Rhamnolipid/Alginat/Pyoverdin-Komplex nach Anspruch 18 oder 19.

30. Dünger oder Düngeradditiv, enthaltend oder bestehend aus
einer Mischung nach einem der Ansprüche 1 bis 15 oder dem supramolekularen Rhamnolipid/Alginat-Komplex nach Anspruch 16 oder 17 oder dem supramolekularen Rhamnolipid/Alginat/Pyoverdin-Komplex nach Anspruch 18 oder 19.

31. Behandelter Pflanzensamen, erhalten oder erhältlich durch
Behandlung von Pflanzensamen mit der Mischung nach einem der Ansprüche 1 bis 15 oder dem supramolekularen Rhamnolipid/Alginat-Komplex nach Anspruch 16 oder 17 oder dem supramolekularen Rhamnolipid/Alginat/Pyoverdin-Komplex nach Anspruch 18 oder 19.

32. Futtermittel oder Futtermittelzusatzstoff, enthaltend
eine Mischung nach einem der Ansprüche 1 bis 15 oder den supramolekularen Rhamnolipid/Alginat-Komplex nach Anspruch 16 oder 17 oder den supramolekularen Rhamnolipid/Alginat/Pyoverdin-Komplex nach Anspruch 18 oder 19.

33. Nahrungsmittel oder Nahrungsmittelzusatzstoff, enthaltend
eine Mischung nach einem der Ansprüche 1 bis 15 oder den supramolekularen Rhamnolipid/Alginat-Komplex nach Anspruch 16 oder 17 oder den supramolekularen Rhamnolipid/Alginat/Pyoverdin-Komplex nach Anspruch 18 oder 19.

34. Pflanzenschutzmittel, insbesondere Insektizid oder Elicitor, oder Pflanzenschutzmitteladditiv, enthaltend
eine Mischung nach einem der Ansprüche 1 bis 15 oder den supramolekularen Rhamnolipid/Alginat-Komplex nach Anspruch 16 oder 17 oder den supramolekularen Rhamnolipid/Alginat/Pyoverdin-Komplex nach Anspruch 18 oder 19.

35. Feuerlöschmittel oder Löschmittelzusatz, enthaltend
eine Mischung nach einem der Ansprüche 1 bis 15 oder den supramolekularen Rhamnolipid/Alginat-Komplex nach Anspruch 16 oder 17 oder den supramolekularen Rhamnolipid/Alginat/Pyoverdin-Komplex nach Anspruch 18 oder 19.

36. Korrosionsschutzmittel, enthaltend
eine Mischung nach einem der Ansprüche 1 bis 15 oder den supramolekularen Rhamnolipid/Alginat-Komplex nach Anspruch 16 oder 17 oder den supramolekularen Rhamnolipid/Alginat/Pyoverdin-Komplex nach Anspruch 18 oder 19.

37. Fettlöser, enthaltend oder bestehend aus
einer Mischung nach einem der Ansprüche 1 bis 15 oder dem supramolekularen Rhamnolipid/Alginat-Komplex nach Anspruch 16 oder 17 oder dem supramolekularen Rhamnolipid/Alginat/Pyoverdin-Komplex nach Anspruch 18 oder 19.

38. Bohrlochflüssigkeit, enthaltend
eine Mischung nach einem der Ansprüche 1 bis 15 oder den supramolekularen Rhamnolipid/Alginat-Komplex nach Anspruch 16 oder 17 oder den supramolekularen Rhamnolipid/Alginat/Pyoverdin-Komplex nach Anspruch 18 oder 19.

39. Verwendung der Mischung nach einem der Ansprüche 1 bis 15 und/oder des supramolekularen Rhamnolipid/Alginat-Komplexes nach Anspruch 16 oder 17 und/oder des supramolekularen Rhamnolipid/Alginat/Pyoverdin-Komplexes nach Anspruch 18 oder 19
für die Herstellung von kosmetischen, dermatologischen oder pharmazeutischen Formulierungen, von Pflanzenschutz-Formulierungen oder von Pflege- oder Reinigungsmitteln oder von Tensidkonzentraten oder bei der Tertiären Erdölförderung oder bei der Erdölraffination oder der Raffination von Pflanzenölen.
